Europäisches Patentamt

European Patent Office

Office européen des brevets

(19)

(11) Publication number: **0 427 875 A1**

# EUROPEAN PATENT APPLICATION
## published in accordance with Art. 158(3) EPC

(21) Application number: 90908664.7

(22) Date of filing: 04.06.90

(86) International application number:
PCT/JP90/00723

(87) International publication number:
WO 90/14790 (13.12.90 90/28)

(51) Int. Cl.⁵: **A61B 5/00, G06F 15/02, G06F 15/20, A61B 10/00**

(30) Priority: 05.06.89 JP 142645/89

(43) Date of publication of application:
**22.05.91 Bulletin 91/21**

(84) Designated Contracting States:
**DE FR GB**

(71) Applicant: **OTSUKA PHARMACEUTICAL CO., LTD.**
**9, Kandatsukasacho 2-chome**
**Chiyoda-ku Tokyo 101(JP)**

(72) Inventor: **MASAKI, Kyosuke**
**2-2-80-901, Nagatoishi Kurume-shi**
**Fukuoka 830(JP)**
Inventor: **MIWA, Sachiko 225-7, Hiraishi**
**Sumiyoshi**
**Kawauchi-cho Tokushima-shi**
**Tokushima 771-01(JP)**
Inventor: **YOSHIOKA, Yasuyuki 2-2-24-301,**
**Nagatoishi**
**Kurume-shi**
**Fukuoka 830(JP)**
Inventor: **KORI, Hideaki**
**6-8, Kogashira-cho Kurume-shi**
**Fukuoka 830(JP)**

(74) Representative: **Ritter und Edler von Fischern, Bernhard,Dipl.-Ing. et al**
**HOFFMANN - EITLE & PARTNER**
**Arabellastrasse 4**
**W-8000 München 81(DE)**

(54) **HEALTH MANAGEMENT SYSTEM.**

(57) Excess or deficiency of nutrition can be judged based on actually ingested foods and behavior of an individual by inputting data on meals ingested by an individual and that on behavior of an individual such as sleeping hours, amount of exercise, etc. and comparing them with nutrition data by food and data of desired values of nutrition suitable to typical behavior and bodily characteristic of an individual stored in advance. Based on this, information on desirable nutrition ingestion and/or exercise can be prescribed.

# Fig. 1

```
        ┌─────────────────────────┐  ①
        │          MENU           │
        └─────────────────────────┘
                     │
        ┌─────────────────────────┐  ②
        │        INPUT DATA        │
        │                          │
        │ (1) PERSONAL BASIC DATA  │
        │ (2) BODY MEASURED DATA   │
        │ (3) CLINICAL EXAMINATION │
        │     DATA                 │
        │ (4) LIFE-STYLE DATA      │
        │ (5) MEAL DATA            │
        └─────────────────────────┘
                     │
        ┌─────────────────────────┐  ③
        │        EDIT DATA         │
        │       SELECT FILE        │
        └─────────────────────────┘
                     │
      ┌──────────────┼──────────────────┐
      ▼              ▼                   ▼
┌────────────┐ ┌────────────┐   ┌──────────────┐
│EVALUATION  │④│EVALUATION  │⑤ │ POSSIBILITY  │⑥
│    OF      │ │    OF      │   │     OF       │
│ NUTRITION  │ │ LIFE-STYLE │   │ ADULT DISEASE│
└────────────┘ └────────────┘   └──────────────┘
```

## HEALTH CONTROL SYSTEM

### Field of the Invention

The present invention relates to a health control system into which entered are (i) the bodily data of a person such as gender, age, body form, bodily strength and the like, (ii) the life-activity data of the person such as sleeping hours, the amount of physical exercise and the like, and (iii) the intake data of the person such as meals, favorites and the like, and which calculates the nutritive balance, the calorific balance of life-style and the like based on comparison of such entered data with the target nutritive data for this person, thereby to indicate the recommended nutrition and life-style.

### Background of the Invention

It has been getting popular to suitably and collectively control meals and physical exercise according to the bodily features of a person, thereby to prevent the person from becoming fat and suffering from a disease of adult people, so that his (her) bodily strength and health are maintained and promoted.

There has been proposed a health examination method comprising the steps of measuring the physical functions of a person such as gender, physical features of body form and the like, bodily strength, the capacity of the lungs and the like, analyzing and medically examining the pulse, the electrocardiogram, the blood, the urine and the like, entering the data thus obtained from a terminal device into a computer, executing in the computer an operation of comparison of the data thus entered with the reference data of a standard healthy person and supplying, based on the comparison results, the potential disease, the optimum prescription of physical exercise and the optimum prescription of meals (See Japanese Unexamined Patent Publication No. 211760/1986). Based on the data thus supplied, it is possible to personally indicate and guide the potential disease, the optimum physical exercise to be taken (such as gymnastics, swimming, jogging or the like) and the optimum meal prescription (such as ideal meal menus, healthy foods or the like). Accordingly, this eliminates the labor of obtaining information through questionnaires and entering individual data into a computer as conventionally done. Also, such a system may automatically indicate a health control method based on the software in the computer, instead of the reliance on judgment of a medical doctor as conventionally done.

In the system above-mentioned, however, if only the data of physical features and physical functions of a person are to be entered, such data cannot be considered as sufficient to control the health of this person.

For example, the contents of meals to be taken at respective occasions by a person are different from one another. At one occasion, a simple meal may be taken and at another occasion, a meal having a high caloric value may be taken. That is, a variety of different nutritive elements are taken by different people. Further, the sleeping hours, the amount of physical exercise and the like of the same person vary from day to day.

Accordingly, if a health control method is determined based on only the information about the physical and medical matters of a person, it is skeptical that such a method would present a prescription suitable for the person. In particular, meals and physical exercise chiefly take part in the prevention of fatness and diseases of adults in which people have taken an increasing interest. It is therefore desired to provide a health control system capable of giving suitable instructions.

It is an object of the present invention to provide a health control system capable of recognizing the excess and deficiency of nutrition and the like based on personal daily data entered thereinto, thereby to give suitable instructions.

### Disclosure of the Invention

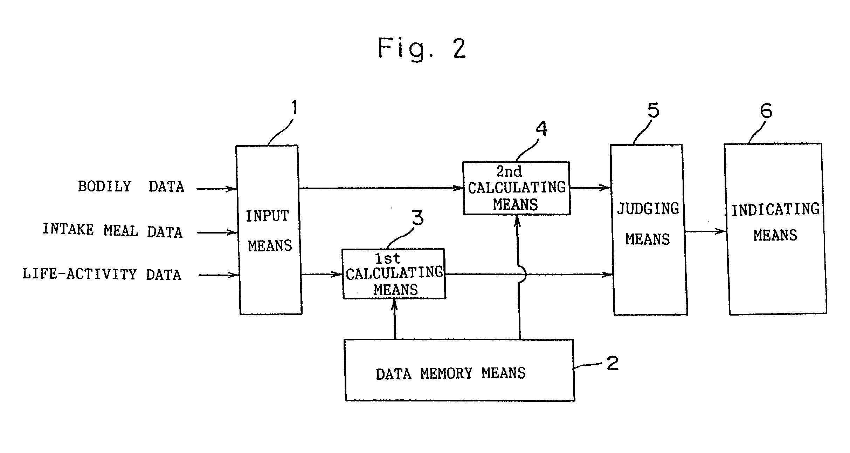

To achieve the object above-mentioned, the health control system in accordance with the present invention comprises, as shown in Fig. 2:

input means 1 for entering the bodily data, intake meal data and life-activity data of a person;

data memory means 2 which previously contains nutrition data per food and also previously contains the target data of nutritive values according to the life-activity, bodily features and the like of a standard person;

first calculating means 3 for calculating, based on the nutrition data contained in the data memory means 2, the intake nutritive values from the intake meal data entered from the input means 1;

second calculating means 4 for correcting, based on the bodily data and the life-activity data entered from the input means 1, the target data contained in the data memory means 2 to calculate the target data of nutritive values for the person;

judging means 5 for judging the excess or deficiency of nutrition by comparing the intake nutritive values calculated by the first calculating means 3 with the target data of nutritive values calculated by the second calculating means 4; and

indicating means 6 for indicating the recommended contents of physical exercise and/or nutrition to be taken based on the judging results supplied by the juding means 5.

In the health control system above-mentioned, the bodily data of a person are entered together with his (her) intake meal data and life-activity data.

The first calculating means 3 calculates the intake nutritive values based on the nutrition data per foods contained in the data memory means 2, and the second calculating means 4 calculates the target data of nutritive values for this person based on the target data of nutritive values contained in the data memory means 2.

Then, the judging means 5 judges the excess or deficiency of nutrition from the difference between the intake nutritive values calculated by the first calculating means 3 and the target nutritive values calculated by the second calculating means 4.

Thus, the excess or deficiency of nutrition may be judged according to the personal specific conditions of intake foods and life-activity.

Based on the judgement results from the judging means 5, the indicating means 6 indicates the recommended contents of nutrition and/or physical exercise to be taken.

As set forth in Claim 2, the indicating means 6 may emphasize the difference between the intake meal data and life-activity data entered by the input means 1 and the recommended contents of nutrition and/or physical exercise to be taken, thereby to indicate the suitable contents of nutrition and/or physical exercise to be taken according to the taste of the person.

As set forth in Claim 3, the clinical examination data of the person may also be entered from the input means 1 and the system may also include estimating means for estimating a potential disease based on the judging results of the judging means 5.

## Brief Description of the Drawings

Figure 1 is a flow chart showing a general procedure of a health control system in accordance with the present invention;

Figure 2 is a functional block diagram for controlling the flow chart in Figure 1; and

Figure 3 is a block diagram of the hardware arrangement of the health control system in accordance with the present invention.

## Preferred Embodiments of the Present Invention

The following description will discuss in detail the present invention with reference to the attached drawings showing an embodiment thereof.

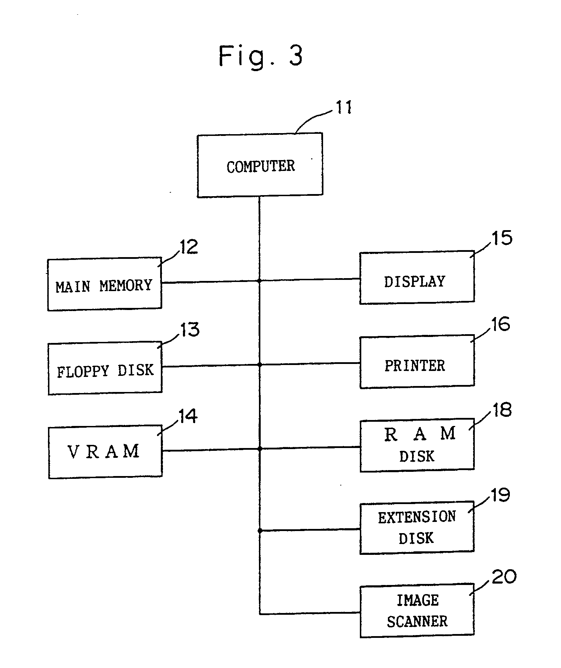

Fig. 3 shows a block diagram of the hardware of the health control system of the present invention. The system includes a computer 11. Connected to the computer 11 through buses are a main memory 12, a floppy disk 13 to which a personal information floppy or a system floppy is to be set, a VRAM 4 for displaying pictures, figures or the like on a display 15, the display 15, a printer 16 for supplying a report of nutrition evaluation, physical exercise evaluation, potential disease and the like, a RAM disc 18 for temporarily storing picture data, an extension disk 19 for storing picture data or a program, and an image scanner 20 for fetching picture data such as an electrocardiogram or the like.

Attached to the display 15 is means (mouse) with which data are entered in an interactive manner.

The personal information floppy stores the basic data, body measured data, clinical examination data, life-style data and meal data of a person. The personal basic data include, in addition to age and gender, data of pregnancy or non-pregnancy, smoker or non-smoker, life-activity intensity (such as the amount of labor) and the like. The body measured data include data of stature, weight, body fat ratio and the like. The clinical examination data include data of blood pressure, cholesterol, blood sugar level and neutral fat. The life-style data include daily data of rising time, bed time and mealtimes, the amount of physical exercise read and entered from a calorie meter mounted on the body, and the number of smoked cigarettes. The

4

meal data include the contents of intake meals.

The extension disk 19 stores the nutritive values and calorie of each dish and also stores RMR (Relative Metabolic Rate) values corresponding to the types of physical exercises (the types of household affairs such as cooking, washing, cleaning and the like, and the types of sports such as swimming, hiking, golf and the like). The extension disk 19 also stores a formula for obtaining a required energy value by adding the basal methabolism to the amount of physical exercise read from the calorie meter, and also stores a calculation table of target nutritive values and a calculation table of target nutritive values for a pregnant woman, in consideration of the consumed energy value according to the types of physical exercises above-mentioned, the personal basic data and the body measured data, and also stores a table for judging the excess or deficiecy of nutritition based on the target nutritive values and the target nutritive values for a pregnant woman above-mentioned.

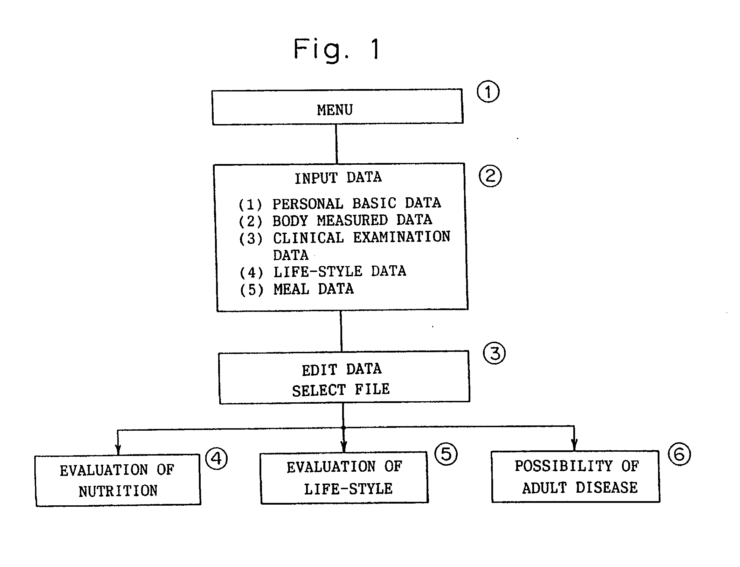

The following description will discuss the general procedure of the health control system with reference to the flow chart in Fig. 1.

The personal basic data, the body measured data, the clinical examination data, the life-style data and the meal data are entered into the personal information floppy by selecting data from the proposed candidates displayed on the display 15 with the use of the mouse or keyboard through which numerals are entered (blocks (1) and (2) in Fig. 1). Such data may be entered every day or collectively for a few days.

As the personal basic data, there are successively entered, with the use of the mouse, the date of birth, gender, pregnancy or non-pregnancy, smoker or non-smoker, target body weight value and life-activity intensity as shown in Table 1. The target intake amounts of nutrition vary with the target body weight value for which the self-weight or standard weight is to be selected. If the standard weight is selected, there are indicated the target intake amounts of nutrition calculated for reducing the body weight.

## Table 1

| Personal Basic Data | | |
|---|---|---|
| Full Name | | |
| Date of Birth | Date | Month | Year |
| Gender | Male | Female |
| Pregmant ? | Yes | No |
| Smoker ? | Yes | No |
| Target Body Weight | Self-weight | Target weight |
| Life-Activity Intensity | Male | Femal |
| Light | Engineer, Public service personnel, Office clerk | Office clerk, Musician, House-wife having no babies |
| Moderate | Medical doctor, Primary school teacher, Manu-facturer, Sales-man | Nurse, Primary school teacher, Housewife having babies |
| Slightly heavy | Agriculture, Fishery, Engaged in con-struction | Health nurse, Engaged in agri-culture |
| Heavy | Engaged in tim-ber-felling & timber trans-portation, Professional sport player | Engaged in agri-culture during the busiest farm-ing season, Professional sport player |

As the body measured data, there are successively entered, with the use of the mouse, stature, body weight, body fat rate, waist size, hip size and measurement date as shown in Table 2.

## Table 2

| Body Measured Data | | | |
|---|---|---|---|
| Stature | | cm | |
| Body Weight | | kg | |
| Body fat rate | | % | |
| Waist size | | cm | |
| Hip size | | cm | |
| Measurement date | Date | Month | Year |

As the clinical examination date, there are successively entered, with the use of the mouse, blood pressure, total cholesterol, HDL cholesterol, blood sugar level, neutral fat and measurement date as shown in Table 3.

## Table 3

| Clinical Examination Data | | | |
|---|---|---|---|
| Blood pressure | to | mmHg | |
| Total cholesterol | | mg/dl | |
| HDL cholesterol | | mg/dl | |
| Blood sugar level | | mg/dl | |
| Neutral fat | | mg/dl | |
| Measurement date | Date | Month | Year |

As the life-style data, there are successively entered data of rising time, bed time and mealtimes, the period of time of physical exercise, the amount of physical exercise read from a calorie meter mounted on the body, and the number of smoked cigarettes. The amount of physical exercise read from the calorie meter refers to that of physical exercise which the person has taken from the rising time to the bedtime.

## Table 4

| Life-Style Data | | |
|---|---|---|
| Bedtime on the previous day: | o'clock | minute |
| Rising time: | o'clock | minute |
| Breakfast: | o'clock | minute |
| Snack in the morning: | o'clock | minute |
| Lunch: | o'clock | minute |
| Snack in the afternoon: | o'clock | minute |
| Dinner: | o'clock | minute |
| Supper: | o'clock | minute |

Physical exercise:
    Morning:    o'clock   minute  to  o'clock    minute
    Afternoon:  o'clock   minute  to  o'clock    minute

| Amount of physical exercise: | kcal |
|---|---|
| Number of smoked cigarettes: | pieces |

Meal data are entered in several steps. For each of breakfast, lunch and dinner, food contents which are most nearly approximated to the really taken ones, are selected as shown in Table 5. Either image input or list input is also selected.

## Table 5

**Meal Data**

Food name
Rice, Rice cake
Bread, Pizza or other
Noodles
Soups
Meat dish
Egg dish
Bean dish, Tofu dish
Fish dish
Vegetable dish
Food served in a pot

Meal to be taken outside
Breakfast
Lunch of fixed menu
Rice meal
Rice meal served in a bowl
Sushi
Bread meal
Buckwheat noodles, Wheat noodles
Spaghetti, Pasta
Chinese food
Box lunch

Dessert, Sweet stuff
Fruit
Dessert on the market
Cakes
Sweet stuff on the market
Ice cream
Japanese sweet stuff
Teahouse, Parlour
Azuki-bean soup with rice cake

Drinks
Milk, Dairy drink
Tea, Coffee
Refreshing drink
Carbonic acid
Teahouse, Parlour
Alcoholic drink
Nutritive drink, Nutritive food

Image Input or List Input ?

When "Image Input" is selected and "Bread, Pizza or other" is then selected, the display 15 displays pictures of butter-roll, toast, sandwiches and the like. Then, the type of the bread taken is entered. For example, when "toast" is entered, the display 15 then displays another screen of pictures showing a slice of toast, a slice of pizza toast, a slice of French toast and the like. The display 15 also displays numerals (1/3, 1/2, 1, 2, 3 times) representing the multiples of amount of the article shown in each picture. One of the pictures and the multiple corresponding to the intake amount are selected and entered. For example, when two slices of pizza toast have been taken, "Pizza toast" and "2" are entered. In addition to the toast picture, there is displayed a selection screen of pictures of coffee, tea, fruit, egg, salad and the like. The foods and amounts concerned are entered. Thus, the operator may intuitively select and enter the intake meals while watching the pictures.

When "List Input" is selected instead of "Image Input" and "Meal of Fixed Menu" is then selected, the display 15 displays a list of a cut-price breakfast served during the morning hours, a hotel breakfast set, a combination set of the buffet type, Japanese meal of fixed menu, morning rice gruel of fixed menu and the like, together with the multiple numerals such as 1/3, 1/2, 1, 2, 3 and the like. When the meal of fixed menu concerned is entered, the contents of the foods (toast, egg, salad, coffee, tea, iced coffee and the like) are displayed in the form of a list. Then, the intake foods and their amounts are entered. At this time, the multiple numerals may be freely changed so that data near the contents of actually taken are entered.

The computer 11 edits the data entered into the personal information floppy and selects, based on the data thus edited, a necessary file from the extension disk 19 (block (3)). Then, the following processing is carried out.

First, the basal methabolism is added to the amount of physical exercise read from the calorie meter to obtain the personal required energy value, and the calories of the intake meals are summed up to obtain the intake energy value. More specifically, the required energy value is obtained as set forth below.

Required energy value per day
$$= 0.9 \times Bm \times Ts + 1.2 \times Bm \times Tr + (\text{Activity Amount})$$
where

| | |
|---|---|
| Bm: | Reference value of basal methabolism per life-activity intensity per day x body weight/24 |
| Ts: | Sleeping hours per day |
| Tr: | Awakening hours per day |
| Activity Amount: | Calory meter reading value x correction coefficient |

The reference value of basal methabolism per life-activity intensity Bm may be obtained from the following Table 6, in which "A", "B", "C" and "D" respectively represent "Light", "Moderate", "Slightly Heavy" and "Heavy" in Table 1.

## Table 6

### Reference Value of Basal Methabolism Bm(Kcal/kg/day)

| Age | Male | | | | Female | | | |
|---|---|---|---|---|---|---|---|---|
| | A | B | C | D | A | B | C | D |
| 20-29 | 23.5 | 24.0 | 24.4 | 24.9 | 22.9 | 23.3 | 23.8 | 24.3 |
| 30-39 | 22.4 | 22.9 | 23.4 | 23.8 | 21.5 | 22.0 | 22.4 | 22.9 |
| 40-49 | 21.8 | 22.2 | 22.7 | 23.1 | 20.7 | 21.1 | 21.5 | 21.9 |
| 50-59 | 21.6 | 22.0 | 22.5 | 22.9 | 20.3 | 20.7 | 21.1 | 21.6 |
| 60-64 | 21.4 | 21.8 | 22.3 | 22.7 | 20.2 | 20.6 | 21.1 | 21.5 |
| 65-69 | 21.2 | 21.6 | 22.1 | 22.5 | 20.3 | 20.7 | 21.1 | 21.5 |
| 70-74 | 21.0 | 21.5 | 21.9 | 22.3 | 20.3 | 20.7 | 21.2 | 21.6 |
| 75-79 | 20.5 | 21.0 | 21.4 | 21.8 | 20.5 | 20.9 | 21.4 | 21.8 |
| 80- | 20.3 | 20.8 | 21.2 | 21.6 | 20.3 | 20.8 | 21.2 | 21.6 |

Such a processing is carried out on data for a several days (for example, a week) to judge the excess or deficiency of energy for the week concerned. For example, when the consumed calorie is 2200 kcal per day and the intake calorie is 3067 kcal per day, it is judged that 767 kcal is excessive per day. Such a judgement may be made per day. It is a matter of course that the judgement results are displayed or printed out.

There are referred the required nutritive values calculated from the calculation table of target nutritive values and the intake nutritive values obtained from the intake meals, to a table for judging the excess or deficiency of nutrition or the like, so that the excess or deficiency of nutrition is judged.

The calculation table of target nutritive values may be arranged as shown in Table 7.

### Table 7

#### Calculation Table of Target Nutritive Values

Protein    1.08 g x body weight (kg)
Correction in terms of life-activity intensity

| | |
|---|---|
| Light | None |
| Moderate | None |
| Slightly Heavy | x 1.18 |
| Heavy | x 1.40 |

Fat/energy ratio
Life-activity intensity

| | |
|---|---|
| Light | 23% (20-25%) |
| Moderate | 23% (20-25%) |
| Slightly Heavy | 28% (25-30%) |
| Heavy | 28% (25-30%) |

| | | |
|---|---|---|
| Calcium | 10 mg x body weight | |
| Iron | Male | 10 mg |
| | Female | 12 mg (10 mg after menopause) |
| Potassium | 4 g (2-4 g) | |
| Vitamin A | 31 IU x body weight (kg) | |
| Vitamin B1 | 0.40 mg/1000 kcal | |
| Vitamin B2 | 0.55 mg/1000 kcal | |
| Vitamin C | 50 mg | |
| Vitamin D | 100 IU | |
| Niacin | 6.6 mg/1000 kcal | |
| Salt | 10 g or less | |
| Dietary fiber | 30 g (20-35 g) | |
| Cholesterol | 600 mg | |

In Table 7, however, correction is made on the values of vitamin B1, vitamin B2 and niacin in view of gender, age and life-activity intensity.

For a pregnant person, the data are corrected according to the periods, i.e., the first-half stage of pregnancy, the second-half stage of pregnancy and a period of lactation as shown in Table 8. The required values of nutrition for a pregnant person are shown with amounts for a light labor added, while the required values of nutrition for a person during a period of lactation are shown with amounts for a moderate labor added.

**Table 8**

Calculation Table of Target Nutritive Values for a Pregnant Woman

|  | First Half | Second Half | Lactation Period |
|---|---|---|---|
| Energy | +150 | +350 | +720 kcal |
| Protein | +10 | +20 | +20 g |
| Fat/Energy Ratio | 28 | 28 | 28 % |
| Calcium | +0.4 | +0.4 | +0.5 g |
| Iron | +3 | +8 | +8 mg |
| Vitamin A | 0 | +200 | +1400 IU |
| Vitamin B1 | 0.40 mg/1000 kcal | | |
| Vitamin B2 | 0.55 mg/1000 kcal | | |
| Vitamin C | +10 | +10 | +40 mg |
| Vitamin D | +300 | +300 | +300 IU |
| Niacine | 6.6 mg/1000 kcal | | |

With the use of the table for judging the excess or deficiency of nutrition, it is judged whether each of nutritive elements is deficient, proper or excessive in seven steps of "deficiency - - -", "deficiency - -", "deficiency -", "proper", "excess +", "excess + +" and "excess + + +".

In more detail, the target value of each nutritive element is obtained based on Tables 7 and 8. Accordingly, the judgment is made based on the differencebetween the actual intake data and the target value. Now, the intake calorie is set to Q. When Q is not less than (target value - 100 kcal) and not greater than (target value + 100 kcal), Q is considered as proper. When Q is not less than (target value - 500 kcal) and not greater than (target value - 100 kcal), Q is considered as "deficiency -". When Q is not less than (target value - 1000 kcal) and not greater than (target value - 500 kcal), Q is considered as "deficiency - -". When Q is not greater than (target value - 1000 kcal), Q is considered as "deficiency - - -".

Judgement of excess or deficiency is also made on protein, fat, salt, calcium, phosphorus, iron, potassium, vitamin A, vitamin D, vitamin B1, vitamin B2, vitamin C, niacin, cholesterol, dietary fiber and the like, in addition to calorie.

The computer 11 further executes a processing for estimating the body weight and the obesity index with the excessive or deficient energy value substituted in a predetermined formula. Where the total consumed energy value for $n$ days is E kcal and the total intake energy value for $n$ days is I kcal, the estimated variation of the body weight for $n$ days $\Delta Bw$ is calculated as follows:

$$\Delta \mathrm{B} w \doteq \frac{I - E}{7500} \ \mathrm{kg}$$

The computer 11 further executes a processing for estimating a potential disease of adult people with the personal basic data, the body measured data, the clinical examination data, the presence or absence of abnormality in the electrocardiogram, the presence or absence of smoking habit and the like substituted in an empirical formula.

To indicate the contents of physical exercise to be taken by the person, there are determined the types and amounts of the physical exercises that the person should take to consume the excessive energy. Such a determination is made based on the RMR values corresponding to the types of physical exercises. The specific RMR values are shown in Table 9.

## Table 9

RMR Values of Physical Exercises

| Types of Household Affairs | | Types of Physical Exercises | |
|---|---|---|---|
| Cooking | 1.6 | Jogging | 6.0 |
| Dish washing | 1.6 | Running | 12.0 |
| Hanging-out & taking-in the washing | 2.2 | Radio gymnastics | 3.5 |
| Hanging-out & taking-in thick quilts | 4.9 | Hiking | 3.0 |
| Ironing | 1.5 | Mountain-climbing | 6.0 |
| Wiping with a cloth | 3.5 | Rope skipping | 8.0 |
| Sweeping & cleaning | 2.2 | Skiing | 6.0 |
| Vacuum cleaner | 1.7 | Cycling | 3.4 |
| Spreading & folding-up thick quilts | 3.5 | Golf (hill) | 5.0 |
| Gardening & weeding | 2.0 | Aerobics | 4.0 |
| Normal walking | 2.1 | Skating | 7.0 |
| Pushing a perambulator | 3.0 | Awa-festival dance | 12.0 |
| Automatic washer | 1.2 | Table tennis | 5.0 |
| Watching TV | 0.2 | Tennis | 6.0 |
| Cleaning a bathroom | 2.4 | Badminton | 6.0 |
| Chattering while seating | 0.2 | Baseball | 2.7 |
| Chattering while standing | 0.3 | Soccer | 7.0 |
| Taking a meal | 0.4 | Football | 7.0 |
| Getup & having a wash | 0.5 | Basket ball | 7.0 |
| Sewing | 0.5 | Volleyball | 6.0 |
| Flower arrangement, mah-jongg, musical instrument | 0.5 | Judo | 6.0 |
| Car driving | 0.5 | Gateball | 2.0 |
| Wordprocessor, making an entry | 0.6 | Bowling | 2.5 |
| Getting on a street-car while standing | 1.0 | Golf (flatland) | 3.0 |
| | | Dancing at disco-theque | 5.0 |
| Shoes polishing | 1.1 | | |
| Bathing | 2.3 | Boat, canoe | 5.0 |
| Babycare | 2.3 | Japanese fencing | 6.0 |
| Walking at a quick pace | 3.5 | Breaststroke | 10.0 |
| Walking at a slow pace | 1.5 | Crawlstroke | 20.0 |
| Going up and down the stairs | 4.6 | Muscle training | 9.6 |

The consumed energy L (kcal) of each physical exercise is obtained according to the following equation:

$$L = RMR \times T \times Bw \times Bm$$

where

T : Period of time of physical exercise (min),

Bm : Reference value of basal methabolism in Table 7 as converted per minute, and

Bw : Body weight

According to the equation above-mentioned, there may be determined the types and periods of time of the physical exercises required to consume the excessive energy.

The data thus obtained are temporarily stored in a VRAM 14 and may be displayed on the display 15, from time to time, as requested by the operator.

When the system is required by the operator to indicate the expected body weight, the expected result of the body weight and obesity index is displayed in the form of a figure, a graph, a picture or the like. When the system is so operated as to indicate the possibility of the person suffering from a disease of adult people, the result is displayed in the form of a graph or the like (block (6)).

When the system is required as to indicate the evaluation of the life-style, there are displayed, for a predetermined number of days, for example a week, data of sleeping hours, periods of time of physical exercises, consumed calorie, basal methabolism value, added amount of physical exercise, intake calories (as classified per each of the meals of breakfast, lunch and dinner), the number of smoked cigarettes, consumed amounts of alcohols such as sake, beer and the like (block (5)). Thus, the operator may evaluate, at a look, his (her) recent life style.

When the system is required to indicate the evaluation of nutrition, the intake nutritive values per nutritive element are displayed with the evaluation of excess or deficiency according to the seven steps above-mentioned also displayed as emphasized (block (4)). For example, when the calorie is ranked as "excess + + +", the salt as "excess + +", the iron as "deficiency - -", the dietary fiber as "deficiency -", such data are displayed in different colors, respectively.

With the use of this screen, the operator may start a game, in an interactive manner, for reconsidering the meals taken on that day. For example, when requiring a prescription to consume the excessive calorie of 867 koal, a predetermined operation is carried out to display the dishes taken on that day in order of intake calorie starting from the highest one.

The operator first tries to consume the calorie by taking physical exercises or doing household affairs. In this connection, he (she) designates the dish desired to be exchanged by physical exercises or household affairs. For example, it is assumed that he (she) drunk three cups of sake representing the intake calorie of 609 kcal. When he (she) designates his (her) desire to exchange such intake calorie for physical exercises or household affairs. The screen is changed so that the system asks either physical exercises or household affairs to be selected. When he (she) selects the physical exercises, the screen is changed to display the types of the physical exercises listed in Table 9, such as runing, football, basket ball, swimming and the like. For each of the types of the physical exercises, there is displayed a period of time required to consume the excessive calorie. The operator may designate one or more types of physical exercises. For example, the operator may designate "swimming for 87 minutes". This means that the intake calorie of 609 kcal generated by the sake is consumed by the physical exercise. It is a matter of course that suitable items may be selected from the household affairs to consume the calorie.

When it is desired to consume the remaining calorie of 258 kcal by reducing the amount of meals rather than by physical taking exercise, the system is accordingly designated. Then, there are displayed the dishes taken on that day in order of calorie starting from the highest one. The operator may select food presenting calorie near 258 kcal, for example, a bottle of beer of 247 kcal. Of course, it is also possible to reduce several foods each in a small amount, instead of single food in a great amount.

When it is desired to change the types of foods, there is displayed a menu of foods having less calorie than that of the food to be exchanged. The operator may designate preferred foods.

Thus, the operator may finally determine, in a manner interactive with the computer, the contents of physical exercises or foods to consume the excessive calorie. The contents thus determined may be printed out by the printer 16.

With the operations above-mentioned, the operator may continuously take, for the future, meals or exercise with clear awareness.

The following description will discuss how to replenish deficient nutritive values. When the deficient nutritive element, for example niacin, is designated,the menu as shown in Table 5 is displayed to display the concentration of niacin in each of the dishes.

When it is desired to replenish the shortage of nutritive value by adding food, the operator may designate preferable food as selected from the displayed items. Then, the replenished amounts of nutritive values are automatically calculated. The operator may continuously designate several foods until the target value is finally reached. If there are displayed foods that the operator does not like, the operator may not designate such foods. If nutrition is excessive, the system may be so used as to reduce the nutrition.

As discussed in the foregoing, to compensate the excessive or deficient calorie and the excessive or deficient nutritive values, the operator may suitably use, in a manner interactive with the computer, the system of this embodiment while suitably increasing or decreasing the contents of meals and physical exercises to be taken according to his (her) preference.

Accordingly, the system may finally display the contents of physical exercises and meals which have calories and nutritive values required for the person and which may be taken with relatively less resistance. Thus, the system of the present invention is excellent in the arrangement to display data of meals in accord with favorite meals that a person concerned generally takes, instead of the arrangement to display an ideal prescription of meals with the personal preference disregarded.

It is also possible for the operator to enhance his (her) knowledge and awareness of nutrition and physical exercise through a dialogue with the system. This serves as an aid to prevent the operator from becoming fat and suffering from diseases of adult people.

According to the health control system of the present invention, the bodily data, intake meal data and life-activity data of a person such as sleeping hours, the amount of physical exercises and the like are entered into the system, so that the system judges excessive or deficient nutritive elements according to the specific contents of his (her) intake meals and life-activity, based on which the system may indicate the contents of meals and/or physical exercises that he (she) is recommended to take. It is therefore possible for him (her) to enhance the knowledge and awareness of nutrition and physical exercise to promote his (her) health.

## Claims

1. A health control system comprising:
   input means for entering the bodily data, intake meal data and life-activity data of a person;
   data memory means which previously contains nutrition data per food and also previously contains the target data of nutritive values according to the life-activity, bodily features and the like of a standard person;
   first calculating means for calculating, based on said nutrition data contained in said data memory means, the intake nutritive values from said intake meal data entered from said input means;
   second calculating means for correcting, based on said bodily data and said life-activity data entered from said input means, said target data contained in said data memory means to calculate the target data of nutritive values for said person;
   judging means for judging the excess or deficiency of nutrition by comparing said intake nutritive values calculated by said first calculating means with said target data of nutritive values calculated by said second calculating means; and
   indicating means for indicating the recommended contents of physical exercises and/or nutrition to be taken, based on the judging results supplied by said juding means.

2. A health control system according to Claim 1, wherein the indicating means indicates the recommended contents of nutrition to be taken, based on comparison of the intake meal data entered from the input means with the judging results of the juding means.

3. A health control system according to Claim 1, wherein the clinical examination data of a person are also entered from the input means, and there is further disposed estimating means for estimating a potential disease based on the judging results of the judging means.

# Fig. 1

```
┌─────────────────────────────────┐
│              MENU               │  ①
└─────────────────────────────────┘
                 │
┌─────────────────────────────────┐
│            INPUT DATA           │  ②
│                                 │
│  (1) PERSONAL BASIC DATA        │
│  (2) BODY MEASURED DATA         │
│  (3) CLINICAL EXAMINATION       │
│      DATA                       │
│  (4) LIFE-STYLE DATA            │
│  (5) MEAL DATA                  │
└─────────────────────────────────┘
                 │
┌─────────────────────────────────┐
│           EDIT DATA             │  ③
│          SELECT FILE            │
└─────────────────────────────────┘
                 │
     ┌───────────┼───────────┐
     ▼           ▼           ▼
┌──────────┐ ┌──────────┐ ┌──────────┐
│EVALUATION│ │EVALUATION│ │POSSIBILITY│
│   OF   ④ │ │   OF   ⑤ │ │   OF    ⑥ │
│NUTRITION │ │LIFE-STYLE│ │ADULT     │
│          │ │          │ │DISEASE   │
└──────────┘ └──────────┘ └──────────┘
```

EP 0 427 875 A1

# Fig. 2

EP 0 427 875 A1

# Fig. 3

# INTERNATIONAL SEARCH REPORT

International Application No **PCT/JP90/00723**

## I. CLASSIFICATION OF SUBJECT MATTER (if several classification symbols apply, indicate all) ⁶

According to International Patent Classification (IPC) or to both National Classification and IPC

Int. Cl⁵    A61B5/00, G06F15/02, G06F15/20, A61B10/00

## II. FIELDS SEARCHED

### Minimum Documentation Searched ⁷

| Classification System | Classification Symbols |
|---|---|
| IPC | A61B5/00, A61B10/00, G06F15/02, G06F15/20 |

Documentation Searched other than Minimum Documentation
to the Extent that such Documents are Included in the Fields Searched ⁸

| | |
|---|---|
| Jitsuyo Shinan Koho | 1926 - 1988 |
| Kokai Jitsuyo Shinan Koho | 1971 - 1989 |

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| Y | JP, A, 64-20829 (Suzuken Co., Ltd.), 24 January 1989 (24. 01. 89) | 1 - 3 |
| Y | JP, A, 62-257567 (Mitsubishi Electric Corp.), 10 November 1987 (10. 11. 87) | 1 - 3 |
| Y | JP, A, 59-188771 (Sekisui Chemical Co., Ltd.), 26 October 1984 (26. 10. 84) | 1 - 3 |
| A | JP, A, 63-262123 (Sharp Corp.), 28 October 1988 (28. 10. 88) | 1 - 3 |
| A | JP, A, 62-293373 (Omron Corp.), 19 December 1987 (19. 12. 87) | 1 - 3 |
| A | JP, A, 62-254728 (Sekisui Chemical Co., Ltd.), 6 November 1987 (06. 11. 87) | 1 - 3 |
| A | JP, A, 61-127061 (Matsushita Electric Works, Ltd.), 14 June 1986 (14. 06. 86) | 1 - 3 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 20, 1990 (20. 08. 90) | September 3, 1990 (03. 09. 90) |

| International Searching Authority | Signature of Authorized Officer |
|---|---|
| Japanese Patent Office | |

Form PCT/ISA/210 (second sheet) (January 1985)